# EUROPEAN PATENT APPLICATION

(11) **EP 4 116 435 A1**
(43) Date of publication of application: **11.01.2023**
(21) Application number: 21382617.5
(22) Date of filing: 08.07.2021
(51) Int. Cl.: C12Q 1/6886

(54) **IN VITRO METHOD FOR THE DIAGNOSIS AND/OR PROGNOSIS OF MALIGNANT MELANOMA**

(71) Applicant: Universidad de Granada, 18071 Granada (ES); Fundación Pública Andaluza Progreso Y Salud, 41092 Sevilla (ES)
(72) Inventor: ÁLVAREZ CUBERO, María Jesús, 18016 Granada (ES); MARCHAL CORRALES, Juan Antonio, 18016 Granada (ES); MARTÍNEZ GONZÁLEZ, Luis Javier, 18016 Granada (ES)
(74) Representative: Hoffmann Eitle

(57) **Abstract**

The present invention refers to an *in vitro* method for the diagnosis and/or prognosis of malignant melanoma (MM). Moreover, the present invention also refers to an *in vitro* method for determining whether a patient suffering from MM is responding to a treatment.

## Description

### FIELD OF THE INVENTION

The present invention refers to the medical field. Particularly, it refers to an *in vitro* method for the diagnosis and/or prognosis of malignant melanoma (MM). Moreover, the present invention also refers to an *in vitro* method for determining whether a patient suffering from MM is responding to a treatment.

### STATE OF THE ART

In 2017, approximately 87,110 individuals were predicted to be diagnosed with MM in the United States; and its incidence rate is increasing around 3-7% with 13/100,000 in European countries. While it still represents less than 5% of all cutaneous malignancies, MM accounts for most skin cancer deaths.

One of the main challenges in this and in many tumors is the need to identify, characterize, and validate informative biomarkers, biomarker patterns, or surrogate markers in order to not only improve early diagnosis of MM but also for differential diagnosis, staging, prognosis, therapy selection, and therapy monitoring. Currently, no ideal biomarker exists for MM diagnosis; just pathological characteristics of the primary melanoma, for example, tumor thickness (Breslow thickness), mitotic rate, and ulceration are now important prognostic factors. In addition, a family history of MM, skin and hair colour, and ultraviolet (UV) light exposure, as well as, altered susceptibility genes regulation, are considered as risk factors for MM. Recent studies suggested the role of several germline variants (MTAP (rs10811629, rs2218220, rs7023329 and rs751173), PLA2G6 (rs132985 and rs2284063), IRF4 (rs12203592), and PAX3 (rs10180903 and rs7600206)) with an association with a low nevus count, certain dermoscopic features and worse prognosis melanoma.

The role of mRNA genomic and epigenomic MM biomarkers in blood and tissue are being also analyzed. Specific genes (gp100, TRP-1, TRP-2, and MART-1) that have functional roles in tumor progression are of interest as mRNA biomarkers in both tissue and blood. As genomic biomarkers, it should be highlighted the role of BRAF (V600E mutation) that has been proved as significantly higher in metastatic tumors. Frequent loss of heterozygosity (LOH) of DNA microsatellites on specific chromosomal regions has also been reported in cutaneous MM. Just to name epigenomic variations, it is included the proved role of the hypermethylation of two regions in the RASSF1A CpG island in metastatic cutaneous MM. Recent studies also suggested the role of several genes induced by exercise and their relation to pathways involved in melanoma, such as ITGB2, WDFY4, ITGAM, CYBB, MMP2, and PARP14. Others suggested the role of TREM2 as a prognostic marker in various malignant tumors, such as MM, because of its role in tumorigenesis and tumor immunity.

According to markers with relevance in therapy, it has been described those alterations in RB1 are the most frequent as a resistance mechanism. The relevance could be marked by the value of RB1 downregulation as an important and frequent alteration implicated in acquired resistance to BRAF inhibitors. For that reason, RB1 is one of the markers that can be adapted and implemented to new targeted therapies. Recent data also suggested the role of miR-181 as a clinically relevant candidate for therapeutic development or for therapy selection.

Furthermore, most of the research is focused on analyzing the molecular signatures of MM for having an early diagnosis and proper monitoring of the disease and treatment. Recent GWAS studies reinforce the role of several melanoma susceptibility genes (MC1R, CDKN2A, TERT, OCA2, and ARNT/SETDB1) but also identified novel top genes with immune and cytokine signalling functions. Mutations in the high-penetrance susceptibility genes such as cyclin dependent kinase inhibitor 2A (CDKN2A) and cyclin dependent kinase 4 (CDK4) occur in 20-40% of familial MM. Alterations in CDKN2A gene are postulated to be involved in DNA repair, apoptosis and invasion by regulating expression of MITF transcription factor; exerting a potential effect on survival. Nevertheless, alterations in these two genes only confer susceptibility in a small proportion of MM cases. Moreover, telomerase reverse transcriptase (TERT) promoter mutations that have emerged as one of the most frequent alterations in MM (from intermediate lesions to in-situ melanomas). TERT is the catalytic component of the telomerase complex with a main role in maintaining the telomere length of chromosomes, cell immortality and in controlling cellular activities; events that gave its importance in cancer development. There are also data that confirm CLPTM1L with a role in uveal melanoma oncogenesis. Moreover, several loci have been reported in combination with TERT. Although CLPTM1L function is not fully understood; it was shown to contribute to RAS-dependent transformation and tumorigenesis by its interaction with phosphoinositide 3-kinase (PIK3CA). However, there are reports also indicating the role of germline variants in the influence of tumour thickness, and that could be the clue for biological processes influencing melanoma growth and invasion.

There are confirmed germline variants as risk variants related to prognosis such as A homozygotes regulator of chromosome condensation 2 (RCC2/PADI6) gene in rs7538876 and TT genotype carriers DLG associated protein 1 (DLGAP1) gene in rs9960018; which are associated with poor prognosis in MM. Poly(ADP-Ribose) polymerase 1 (PARP1) gene variants such as rs2249844 (G>A) and rs3219090 are associated with disease outcome, mainly with reduced MM specific survival [19]. Moreover, recent data also reported the impact of BRCA1-associated protein 1 (BAP1) loss/inactivation as a germline mutation that predispose to cutaneous squamous cell carcinoma in situ and invasive squamous cell carcinoma.

Some studies have shown that providing the non-essential aromatic amino acid L-tyrosine to melanoma or melanocyte cell lines will produce an increase in MM cells. On the other hand, L-tyrosine and L-DOPA are essential molecules for the synthesis of pigments in normal melanocytes and carcinogens. They both intervene in the formation of melanosomes, and therefore play an important role in mediating cellular processes affecting melanocyte and its respective receptor cells. Tyrosinase (TYR) is a copper-dependent enzyme that catalyzes the conversion of L-tyrosine into L-DOPA, which is the limiting stage in melanin synthesis ratio. Thus, there are also suspicious that TYR gene could have an important role in melanoma; mainly due to its role as encoding the TYR enzyme. Moreover, two common variants (S192Y and R402Q) are significant contributors to pigmentation variation in European populations. Similarly happens with tyrosinase related protein 1 (TYRP1) gene, which has relevance in making the tyrosinase-related protein 1 enzyme; located in melanocytes. The variant rs2733832 has been significantly associated with multiple primary melanoma.

Several GWAS highlight the role of CTXND2 (rs7412746) as candidate gene for predisposing increased risk of developing melanoma, making a significant influence in the expression of several genes in the region, as well as other GWAS reinforce the role of methylthioadensine phosphorylase (MTAP, rs7023329) in MM and several tumors. The etiology of MM could be divided into a chronic sun-exposure pathway or a nevus pathway; so, at genetic level, pigmentation genes such as MC1R are relevant and others such as MTAP. MX2 has also been discovered with GWAS analysis as a promising protein classified as dynamin like GTPase2. Moreover, there are also proved that altered expression patterns of MX2 gene are altered in MM, suggesting that it can act as a tumor suppressor in melanoma.

Many recent reports try to find genes that could be implicated in prognosis prediction such as the identification of ten-gene signature (SIRT3, HMCES, SLC44A3, TCTN1, STPG1, POMGNT2, RNF208, ANXA2P2, ULBP1 and CA12) with new treatment insights. Or even a deep analysis of the tumor microenvironment for discovering relation to immune system or melanoma biomarkers in CLEC4A, GBP4 and KIR2DL4 genes. However, there are scarce recent data about relevant genetic biomarkers in MM aggressiveness.

So, considering the complexity of current status of germline mutations in MM, there is an unmet medical need of finding reliable biomarkers which can be used for the diagnosis and/or prognosis of MM, or even for determining whether a patient suffering from MM is responding to a treatment. The present invention is thus focused on solving this problem and an *in vitro* method for the diagnosis and/or prognosis of MM, and also for determining whether a patient suffering from MM is responding to a treatment, is herein provided.

### DESCRIPTION OF THE INVENTION

### Brief description of the invention

As explained above, the present invention refers to an *in vitro* method for the diagnosis and/or prognosis of MM. Moreover, the present invention also refers to an *in vitro* method for determining whether a patient suffering from MM is responding to a treatment.

Particularly, the inventors of the present invention have carried out a genetic analysis looking for the role of several non-related single nucleotide polymorphisms (SNPs) including CLPTM1L (rs401681), LOC107987026 (rs1335510), TYRP1 (rs2733832), MTAP (rs7023329), TYR (rs1126809), MX2 (rs45430), CTXND2 (rs7412746) and PARP1 (rs3219090) in the risk and aggressiveness of MM. Moreover, the results provided in the present invention indicate that all the SNPs included in the meta-analysis are promise markers for MM progression and confirm the role played by the TYR enzyme, and polymorphisms in its gene, in the development of melanoma. Bioinformatics, in silico and functional analysis demonstrated that high expression patterns of TYR and TYRP1 genes increase MM aggressiveness. Moreover, meta-analysis offers a high strength and consistence of statistic values in these SNPs. In addition, rs401681 (CLPTM1L), rs1126809 (TYR), rs1335510 (LOC107987026), rs2733832 (TYRP1), rs3219090 (PARP1) and rs7412746 (CTXND2) have been related with risk allele and MM status. So, using these genetic markers in MM management could improve precision medicine in these patients and their close relatives.

Additionally, these data show how important it would be to perform the genotyping of these SNPs in descendants of patients carrying several risk alleles, which would lead to a better follow-up and approach precision medicine in MM.

So, the first embodiment of the present invention refers to an *in vitro* method (hereinafter "method of the invention") for the diagnosis and/or prognosis of MM which comprises assessing, in a biological sample obtained from a subject, the presence of at least one allele of the single nucleotide polymorphism (SNP) rs401681 of gene CLPM1L, or the level of expression of the gene CLPM1L having the SNP rs401681, wherein the presence of the SNP rs401681 of gene CLPM1L, or the identification of a higher level of expression of the gene CLPM1L having the SNP rs401681 as compared with a pre-established threshold value, is an indication that the subject is suffering from MM or has a bad prognosis. The method of the invention is also directed to the determination whether a patient suffering from MM is responding to a treatment which comprises assessing, in a biological sample obtained from the patient, the presence of at least one allele of the SNP rs401681 of gene CLPM1L, or the level of expression of the gene CLPM1L having the SNP rs401681,after the administration of the treatment, wherein the presence of the SNP rs401681 of gene CLPM1L, or the identification of a higher level of expression of the gene CLPM1L having the SNP rs401681 as compared with a pre-established threshold value, after the administration of the treatment, is an indication that the subject is not responding to the treatment.

Moreover, any the following SNPs, or any combination thereof, preferably in combination with the SNP rs401681 of gene CLPM1L, can be used in the context of the present invention: rs3219090 of gene PARP1, rs2733832 of gene TYRP1, rs1126809 of gene TYR, rs1335510 of gen LOC107987026, and/or rs7412746 of gene CTXND2, or the group consisting of: rs3219090 of gene PARP1, rs2733832 of gene TYRP1, rs1126809 of gene TYR, rs1335510 of gen LOCI07987026, and rs7412746 of gene CTXND2. So, in a preferred embodiment, the method of the invention further comprises assessing the presence of at least one of the following SNPs, or determining the level of expression of the genes having the following SNPs: rs3219090 of gene PARP1, rs2733832 of gene TYRP1, rs1126809 of gene TYR, rs1335510 of gen LOC107987026, and/or rs7412746 of gene CTXND2, or the group consisting of: rs3219090 of gene PARP1, rs2733832 of gene TYRP1, rs1126809 of gene TYR, rs1335510 of gen LOC107987026, and rs7412746 of gene CTXND2.

On the other hand, the level of expression of TYR and/or TYRP1 can be used in the context of the present invention alone or in combination with any of the above cited SNPs, preferably in combination with the SNP rs401681 of gene CLPM1L. So, in a preferred embodiment, the method of the invention further comprises determining the level of expression of TYR and/or TYRP1 in a biological sample obtained from the patient, wherein the identification of a higher level of expression as compared with a pre-established threshold value is an indication that the patient is suffering from MM, has a bad prognosis or is not responding to the treatment.

In a preferred embodiment, the diagnosis and/or prognosis of MM carried out according to the method of the invention is confirmed by image techniques, preferably by dermatoscopy.

In a preferred embodiment, the biological sample is skin tissue, saliva, blood, urine, serum or plasma.

In a preferred embodiment, the presence of the SNPs is determined by polymerase chain reaction (PCR), preferably real time/quantitative PCR (qPCR) and the level of expression is determined by real time/quantitative PCR (qPCR).

The second embodiment of the present invention refers to the *in vitro* use of the SNP rs401681 of gene CLPM1L, or of the gene CLPM1L, for diagnosis and/or prognosis of MM, or for determining whether a patient suffering from MM is responding to a treatment.

In a preferred embodiment, the present invention refers to the *in vitro* use of at least one of the following SNPs or one of the genes having the following SNPs: rs1126809 of gene TYR, rs7412746 of gene CTXND2, rs1335510 of gen LOC107987026, rs2733832 of gene TYRP1 and/or rs3219090 of gene PARP1, preferably in combination with the SNP rs401681 of gene CLPM1L, for diagnosis and/or prognosis of MM, or for determining whether a patient suffering from MM is responding to a treatment.

In a preferred embodiment, the present invention refers to the *in vitro* use of the level of expression of TYR and/or TYRP1, preferably in combination with the SNP rs401681 of gene CLPM1L, for diagnosis and/or prognosis of MM, or for determining whether a patient suffering from MM is responding to a treatment.

In a preferred embodiment, the present invention refers to the *in vitro* use of the above cited SNPs or genes, in combination with image techniques, preferably with dermatoscopy, for the diagnosis and/or prognosis of MM, or for determining whether a patient suffering from MM is responding to a treatment.

The third embodiment of the present invention refers to a kit consisting of reagents for the identification of the following SNPs or for assessing the level of expression of genes having the following SNPs: rs401681 of gene CLPM1L, rs1126809 of gene TYR, rs7412746 of gene CTXND2, rs1335510 of gen LOC107987026, rs2733832 of gene TYRP1 and rs3219090 of gene PARP1.

The fourth embodiment of the present invention refers to the use of the kit for the diagnosis and/or prognosis of MM, or for determining whether a patient suffering from MM is responding to a treatment.

The fifth embodiment of the present invention refers to an anticancer therapy including surgery, radiation therapy, chemotherapy (Dacarbazine, Temozolomide, Nab-paclitaxel, Paclitaxel, Cisplatin, Carboplatinimmune), checkpoint inhibitors such as Pembrolizumab, nivolumab, Atezolizumab, or Ipilimumab, interleukin-2 (IL-2), Oncolytic virus therapy such as Talimogene laherparepvec, BCG vaccine, targeted therapy drugs such as BRAF inhibitors (Vemurafenib, dabrafenib or encorafenib), MEK inhibitors (trametinib, cobimetinib or binimetinib), imiquimod cream, imatinib and nilotinib among others, for use in a method for treatment of MM in patients who have been diagnosed by means of the method of the invention explained above.

The sixth embodiment of the present invention refers to a computer program or a computer-readable media containing means for carrying out the method of the invention.

The present invention also refers to other specific embodiments explained below.

Particularly, the present invention refers to method for detecting the presence of a SNP or the level of expression of a gene in a test sample from a human subject at risk of developing MM, the method comprising: a) contacting the test sample with a primer specific to the SNP or the gene, wherein the SNP is selected form the list comprising: CLPTM1L (rs401681), LOC107987026 (rs1335510), TYRP1 (rs2733832), MTAP (rs7023329), TYR (rs1126809), MX2 (rs45430), CTXND2 (rs7412746) and/or PARP1 (rs3219090), or the gene is TYR and/or TYRP1, b) amplifying in order to produce an amplification product in the test sample; and c) determining the presence of the SNPs or the level of expression of the gene by assessing the level of the amplification product in the test sample. So, this method is carried out by using polymerase chain reaction (PCR), preferably real time/quantitative PCR (qPCR).

The present invention also refers to a method for analysing a biological sample prior to diagnosis MM comprising analysing a biological sample for determining the presence of a SNP selected from the following list, or the level of expression of a gene from the following list: CLPTM1L (rs401681), LOC107987026 (rs1335510), TYRP1 (rs2733832), MTAP (rs7023329), TYR (rs1126809), MX2 (rs45430), CTXND2 (rs7412746) and/or PARP1 (rs3219090), or the level of expression of TYR and/or TYRP1, wherein the presence of a least one of these SNPs or the identification of a higher level of expression of at least one of these genes is a indication of MM.

The present invention also refers to a method for treating a patient who has been diagnosed as suffering from MM by using the method of the present invention. This method comprises the administration of an anticancer therapy aimed at treating MM, such as radiation therapy, chemotherapy (Dacarbazine, Temozolomide, Nab-paclitaxel, Paclitaxel, Cisplatin, Carboplatinimmune), checkpoint inhibitors like Pembrolizumab, nivolumab, Atezolizumab, or Ipilimumab, interleukin-2 (IL-2), Oncolytic virus therapy such as Talimogene laherparepvec, BCG vaccine, targeted therapy drugs such as BRAF inhibitors (Vemurafenib, dabrafenib or encorafenib), MEK inhibitors (trametinib, cobimetinib or binimetinib), imiquimod cream, imatinib and nilotinib, among others.

The present invention can be a computer-implemented invention, wherein a processing unit (hardware) and a software are configured to:
- Receive information regarding the presence or level of expression of any of the above cited SPNs or genes,
- Process the information for finding substantial variations or deviations, and
- Provide an output through a terminal display of the variations or deviations found, which indicates that the subject may be suffering from MM

For the purpose of the present invention the following terms are defined:
- The expression "pre-established threshold value" refers to the level observed in patients who are not suffering from MM or has a good prognosis. A patient is likely to suffer from MM with a given sensitivity and specificity if the level of the biomarkers in the patient are above said "pre-established threshold value". Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value must be determined in order to obtain the optimal sensitivity and specificity according to the function of the test and the benefit/risk balance (clinical consequences of false positive and false negative).
- The term "comprising" means including, but not limited to, whatever follows the word "comprising". Thus, use of the term "comprising" indicates that the listed elements are required or mandatory, but that other elements are optional and may or may not be present.
- By "consisting of" it is meant including, and limited to, whatever follows the phrase "consisting of". Thus, the phrase "consisting of" indicates that the listed elements are required or mandatory, and that no other elements may be present.

### Description of the figures

Figure 1. Effect of TYR and TYRP1 expression level on survival was calculated using skin cutaneous melanoma (SKCM) dataset from the Cancer Genome Atlas (TCGA). Patients are classified in two groups according to expression values of TYR and TRYP1 genes. High expression patient shows expression value >3rd quartile, in both analyses. Significance of survival impact (p) is measured by log rank test.
Figure 2. Gene expression level of TYR and TYRP1 depending on rs1126809 and rs2733832 genotype, respectively. TYR: n=12, p-value=0.0133; TYRP1: n=10, p-value=0.7363.
Figure 3. STRING network with the genes of present study.
Figure 4. Forest plot of meta-analysis representing sensibility in rs401681 (CLPTM1L).
Figure 5. Forest plot of meta-analysis in rs1126809 (TYR).
Figure 6. Forest plot of meta-analysis in rs1335510 (LOC 107987026).
Figure 7. Forest plot of meta-analysis in rs2733832 (TYRP1).
Figure 8. Forest plot of meta-analysis in rs3219090 (PARP1).
Figure 9. Forest plot of meta-analysis in rs7412746 (CTXND2).

### Detailed description of the invention

The present invention is illustrated by means of the Examples set below without the intention of limiting its scope of protection.

### Example 1. Materials and Methods

### Example 1.1. Patients

The study included patients with primary melanoma (n=103) from Dermatology Service of both University Hospital Virgen de las Nieves and University Hospital San Cecilio recruited between 2015 and 2017. Demographic (age at diagnosis, sex) and clinical information (disease stage, Breslow thickness, Clark level, mitosis rate (mitoses/mm²), ulceration, histology and primary tumor anatomic site) as well as blood samples from melanoma patients were collected, anonymized and stored at -80°C by the biobank of the Public Health Service of Andalusia (SSPA in Spanish) (See details in **Table 1**). The use of biological samples was approved by the ethical committee from both hospitals (number: 32140085), according to the Helsinki Declaration. Individuals with no evidence of melanoma or another type of cancer (n=117) were used as controls by a dataset store in our scientific group. All participants provided written informed consent.

**Table 1. Demographic and clinical characteristics of study population.**

| | | **Controls** | **Melanoma Patients** |
|---|---|---|---|
| | | **n (%)** | **n (%)** |
| **Sex** | Men | 49 (41.5) | 41 (39.8) |
| | Women | 67 (56.8) | 62 (60.2) |
| | Missing | 1 (1.7) | - |
| **Age** | Range | 29-79 years | 27-86 years |
| | Mean ± SD | 54.66 ± 7.11 years | 55.97 ± 15.68 years |
| **Stage** | 0 (*in situ*) | - | 4 (3.9) |
| | I | - | 26 (25.2) |
| | II | - | 23 (22.3) |
| | III | - | 36 (35.0) |
| | IV | - | 14 (13.6) |
| **Breslow thickness (mm)** | Mean ± SD | - | 2.66 ± 2.56 |
| | ≤ 1.5 | - | 39 (37.9) |
| | > 1.5 | - | 44 (42.7) |
| | Missing | - | 20 (19.4) |
| **Clark level** | I | - | 4 (3.9) |
| | II | - | 6 (5.8) |
| | III | - | 24 (23.3) |
| | IV | - | 41 (39.8) |
| | V | - | 5 (4.9) |
| | Missing | - | 23 (22.3) |
| **Mitotic rate (mm2)** | Mean ± SD | - | 4.06 ± 4.77 |
| | ≤ 3.0 | - | 44 (42.7) |
| | > 3.0 | - | 25 (24.3) |
| | Missing | - | 34 (33.0) |
| **Ulceration** | Yes | - | 23 (22.3) |
| | No | - | 44 (42.7) |
| | Missing | - | 36 (35.0) |
| **Histology** | Superficial | - | 45 (43.7) |
| | Nodular | - | 24 (23.3) |
| | Lentigo | - | 10 (9.8) |
| | Others | - | 2 (1.9) |
| | Missing | - | 22(21.4) |
| **Anatomical Site** | Head/neck | - | 18 (17.5) |
| | Trunk | - | 37 (35.9) |
| | Extremities | - | 38 (36.9) |
| | Missing | - | 10 (9.7) |

| | | | |
|---|---|---|---|
| *Footnote Table 1: Abbreviations: standard derivation (SD)* | | | |

### Example 1.2. DNA extraction

Genomic DNA extraction was obtained from whole blood of patients, by using organic extraction protocols with phenol/chloroform/isoamyl alcohol and proteinase K, followed by purification with Microcon^{®} DNA Fast Flow Filter H 100 filters (Millipore, Germany). Obtained DNA was quantified by the spectrophotometer NanoDrop^{™} 2000c (ThermoFisher Scientific, Waltham, MA, USA) and stored at -20°C until genotyping.

### Example 1.3. Genotyping

We included 8 SNPs: rs401681 (C__1150767_20) in *CLPTM1L* gene; rs1335510 (C__1807834_10) in *LOC107987026* gene; rs2733832 (C__3119210_10) in TYRP1 gene, rs45430 (C__2564407_10) in *MX2* gene; rs7023329 (C29146385_10) in *MTAP* gene; rs1126809 (custom TaqMan^{®} SNP) in *TYR* gene, rs7412746 (C__1809948_10) in *CTXND2* gene and rs3219090 (C__30564104_10) in *PARP1* gene. These genes are involved in risk, progression or aggressiveness of melanoma; SNPs in these genes were selected using The National Center for Biotechnology Information website (https://www.ncbi.nlm.nih.gov/, accessed on 11 June 2021).

DNA genotyping was performed using TaqMan^{®} Genotyping Master Mix (Applied Biosystems, USA) which included all essential components (except probes, templates and water) for polymerase chain reaction (PCR). Allelic discrimination assays were carried out in a 7900HT Fast Real-Time PCR System (Applied Biosystems, USA).

### Example 1.4. TCGA Analysis

In order to know the gene expression patterns during the evolution in advanced melanoma, we have studied the Skin Cutaneous Melanoma (SKCM) dataset from TCGA (The Cancer Genome Atlas). We have analyzed 411 cases (256 males and 155 females) with recording data of clinical stage (stage I (n=77; i, ia, ib); stage II (n=139; ii, iia,iib, iic); stage III (n=170; iii, iiia, iiib,iiic) and stage IV (n=23)) using an online interactive tool, UALCAN, to explore expression profile of our gene of interest and calculating survival profiles classifying patient according to expression values of our target genes (http://ualcan.path.uab.edu). We have developed an analysis of gene expression in all genes except *LOC107987026* and *CTXND2,* and we have compared high expression and low/medium expression patients after classifying the patients into quartiles according to target gene expression (comparing samples in Q1 vs Q2, Q3 and Q4.). Significance of survival impact was measured by log rank test.

### Example 1.5. Functional analysis validation using expression analysis

After genotyping 112 controls from the GENYO sample collection without previous familiar data of melanoma, we called 47 participants to obtain fresh tissue from skin at the base of the fingernails, eponychium. Tissue samples from the donors were collected and preserved in RNAlater (Invitrogen) at -20°C. RNA extraction was performed using RNeasy Plus Universal Mini Kit (QIAcube/QIAGEN) following manufacturer instructions. After RNA extraction, samples were quantified using NanoDrop^{™} 2000c (ThermoFisher Scientific, Waltham, MA, USA) and preserved at -80°C.

A total of 34 of these samples were selected, according to their genotype, for gene expression analysis, which was performed through TaqMan^{®} Gene Expression Assay kit (Applied Biosystems) using *TYR* (Hs00165976) and *TYRP1* (Hs00167051) with *GAPDH* (Hs02758991) probes. Each plate contained an NTC (non-template control) for each single probe. *GAPDH* was used as housekeeping for *TYR* and.

### Example 1.6. In silico analysis on the functional and structural impact of the SNPs

An analysis of the different variants of present study was carried out in "The variant effect predictor" (https://www.ensembl.org/info/docs/tools/vep/index.html, accessed on 11 June 2021). This software was used to calculate changes in transcripts and malignancy of variants. We also used ClinVar tool (https://www.ncbi.nlm.nih.gov/clinvar/, accessed on 11 June 2021) for data validation.

A functional analysis of genes associated with risk was performed: *(PARP1, TYR, TYRP1, CLPTM1L* and *CTXND2).* Moreover, we have also included *IFNE* and *MTAP,* not for their association with melanoma, but for being binding sites of codifying RNA of *LOC107987026* gene. This analysis was performed using IPA (Ingenuity Pathway Analysis) and DAVID Bioinformatics Resources v6.8 (https://david.ncifcrf.gov/, accessed on 11 June 2021) to obtain the role of gene pool, clinical implication, ontology and involved metabolic pathways. Furthermore, STRING search tool was used to calculate Interacting Genes with our target and interaction among our target genes (https://string-db.org/, accessed on 11 June 2021).

### Example 1.7. Meta-analysis

This meta-analysis was performed using the selected SNPs analyzed in our cohort (as it is mentioned in previous section, Genotyping). The search was developed by using several websites such as https://pubmed.ncbi.nlm.nih.gov, accessed on 11 June 2021, https://www.snpedia.com/, accessed on 11 June 2021, and https://www.ebi.ac.uk/, accessed on 11 June 2021. Data were filtered using the following inclusion criteria: a) At least one of the SNPs was related with MM, b) only articles with case-control data. We excluded reports with no data of allelic frequencies in the study.

Afterwards, depending on the number of studies that we found for each SNPs we followed a different way of processing. When SNPs with at least 3 studies (rs1126809, rs1335510, rs2733832, rs3219090, rs401681 and rs7412746) we used MetaGenyo platform (http://bioinfo.genyo.es/metagenyo/, accessed on 11 June 2021). This platform reports statistical data among different genetic variants and melanoma aggressiveness.

### Example 1.8. Statistical analysis

Hardy-Weinberg equilibrium (HWE) and Linkage disequilibrium (LD) analyses were performed using the online SNPstats and Haploview software. Software package SPSS v.22 (IBM Corporation, USA) and Metagenyo platform were used for statistical analyses. The association between MM risk as well as clinical stage and SNPs were analyzed by chi-square a binary logistic regression (adjustment of age and sex covariates) tests using different genetic models (codominant, dominant and recessive). Statistical signification was considered with p-values ≤ 0.05.

### Example 2. Results

### Example 2.1. Study subjects

In this study, 220 individuals, 103 MM patients and 117 controls have been deeply characterized. MM patients' age range was 27-86 years with 55.97 years as median age vs. controls (29-79 years with median 54.11 years). Sex's frequencies were similar in MM cases and controls. However, the prevalence of MM was higher in women (60.2%) compared to men (39.8%). Missing data was due to several reasons: primary melanoma was not known; anatomical site of primary melanoma prevented pathological study; or the clinical characteristics of patients were absent. Subsequently, sample size was increased reaching 6,089 cases and 45,211 controls (n=51,300) for meta-analysis.

### Example 2.2. SNPs and MM Risk Association

All SNPs were successfully genotyped in the total cohort (n=220). SNPs frequencies in patients and controls were in concordance with Hardy-Weinberg equilibrium. Evidence of linkage disequilibrium (LD) was found between rs1335510 and rs7023329 in chromosome 9 (r²=0.5000, D'=0.8808, p-value=3.00^{∗}10⁻¹⁶), matching with the calculation performed in ensembl genome browser, release 104, with 1000 Genomes populations (r²=0.5924, D'=0.9519 in Iberian population). No association was found between rs1335510 - rs7023329 and the other SNPs located at chromosome 9 (rs2733832); or those located at chromosome 1 (rs7412746; rs3219090).

Firstly, we analyzed the association of each SNP with MM risk. **Table 2** summarizes the SNPs genotypes distribution patients and controls. Any SNPs showed statistical differences between both groups.

**Table 2. SNPs genotypes distribution in MM cases and controls.**

| **SNP** | **Controls n (%)** | **Melanoma Cases n (%)** |
|---|---|---|
| **rs401681** (***CLPTM1L*** | | |
| **CC** | 41 (35.0) | 27 (26.2) |
| **CT** | 49 (41.9) | 50 (48.5) |
| **TT** | 27(23.1) | 26 (25.3) |
| **rs1335510 (*LOC107987026*)** | | |
| **TT** | 43 (36.8) | 32 (31.1) |
| **TG** | 53 (45.3) | 53 (51.4) |
| **GG** | 21 (17.9) | 18 (17.5) |
| **rs2733832 (*TYRP1*)** | | |
| **TT** | 28 (23.9) | 26 (25.2) |
| **TC** | 61 (52.2) | 59 (57.3) |
| **CC** | 28 (23.9) | 18 (17.5) |
| **rs45430 (*MX2*)** | | |
| **TT** | 44 (37.9) | 33 (32.0) |
| **TC** | 50 (43.1) | 49 (47.6) |
| **CC** | 22 (19.0) | 21 (20.4) |
| **rs7023329 (*MTAP*)** | | |
| **AA** | 25 (21.4) | 24 (23.3) |
| **AG** | 62 (53.0) | 48 (46.6) |
| **GG** | 30 (25.6) | 31 (30.1) |
| **rs1126809 (*TYR*)** | | |
| **GG** | 69 (58.1) | 51 (49.5) |
| **GA** | 40 (34.2) | 45 (43.7) |
| **AA** | 9(7.7) | 7 (6.8) |
| **rs7412746 (*CTXND2*)** | | |
| **TT** | 28 (23.9) | 33 (32.0) |
| **TC** | 63 (53.8) | 44 (42.7) |
| **CC** | 26 (22.3) | 26 (25.3) |
| **rs3219090 (*PARP1*)** | | |
| **CC** | 52 (44.4) | 53 (51.5) |
| **CT** | 49 (41.9) | 41 (39.8) |
| TT | 16(13.7) | 9 (8.7) |

### Example 2.3. Association between variants and clinical status

Secondly, we analyzed the correlation between SNPs and clinical status of MM patients. We found that rs7023329 *(MTAP)* was significantly associated with disease state. GG genotype (OR=2.556 (1.063-6.143), p=0.036) confer risk of being in high MM stages (III+IV stages). In addition, GG carriers for rs7023329 show a higher Breslow thickness (OR=3.130 (1.151-8.512), p=0.025) and show a tendency of association with a higher mitotic index (OR=2.843 (0.992-8.150), p=0.052).

On the other hand, some of the genetic variants showed an association with the anatomical site of the tumor. Specifically, TG and GG for rs1335510 *(LOC107987026)* (OR=2.783 (1.095-7.070), p=0.031) and TC and CC genotypes for rs2733832 (TYRP1) (OR=2.704 (1.030-7.098), p=0.043) confer risk of developing MM in head, neck or extremities. rs7023329 was near to the association with the anatomical site (see **Table** 3). No association was found for the rest of clinical variables.

**Table 3. Associations between genetics variants and clinical status.**

| **Clinical Variable** | **n** | **SNP** | **Genotypes** | **OR(95% CI)** | **p-value** |
|---|---|---|---|---|---|
| **Melanoma stage** | 103 | rs7023329 (*MTAP*) | *AG+AA* / **GG** | 2.556 (1.063-6.143) | 0.036 |
| **Low-median risk vs. high risk** | | | ***Recessive model*** | | |
| **Breslow thickness (mm)** | 83 | rs7023329 (*MTAP*) | *AG+AA* / **GG** | 3. 130 (1.151-8.512) | 0.025 |
| **≤ 1.5 vs.** > **1.5** | | | ***Recessive model*** | | |
| **Mitotic Index** | 69 | rs7023329 (*MTAP*) | *AG+AA* / **GG** | 2.843 (0.992-8.150) | 0.052 |
| **≤ 3.00 vs. >3.00** | | | ***Recessive model*** | | |
| **Anatomical Site** | | rs7023329 (*MTAP*) | *AA l **AG+GG*** | 2.917 (0.982-8.663) | 0.054 |
| | | | ***Dominant model*** | | |
| | 93 | rs1335510 (*LOC107987026*) | *TT* / ***TG+GG*** | 2.783 (1.095-7.070) | 0.031 |
| T**runk vs Head-Neck- Extremities** | | | ***Dominant model*** | | |
| | | rs2733832 (*TYRP1*) | *TT* / ***TC+CC*** | 2.704 (1.030-7.098) | 0.043 |
| | | | ***Dominant model*** | | |
| **Histology** | 81 | rs2733832 (*TYRP1*) | *TT* / ***TC+CC*** | 4.674 (1.425-15.335) | 0.011 |
| **Superficial vs. rest of histology subtypes** | | | | | |
| | | | ***Dominant model*** | | |

| | | | | | |
|---|---|---|---|---|---|
| *Footnote Table 3.* * *Binary logistic regression adjusted by age and sex. For binary logistic regression melanoma stages were classified in two groups: Low-medium risk melanoma (In situ*+*I*+*IIstages) vs. High-risk melanoma (III+IV stages)* | | | | | |

Finally, haplotypes analysis by position or function was carried out. Present study did not reveal any association in SNPs located at chromosome 1 or 9; or those located in genes involved in melanin synthesis pathways. In chromosome 9 the SNPs in LD, rs1335510 and rs7023329, did not show association with a greater predisposition to cancer development, the haplotypes T-A (44.92%) in our cohort or T-G (13.27%) showed similar risk values than the other two haplotypes (OR (95% CI)≃1).

### Example 2.4. TCGA Analysis

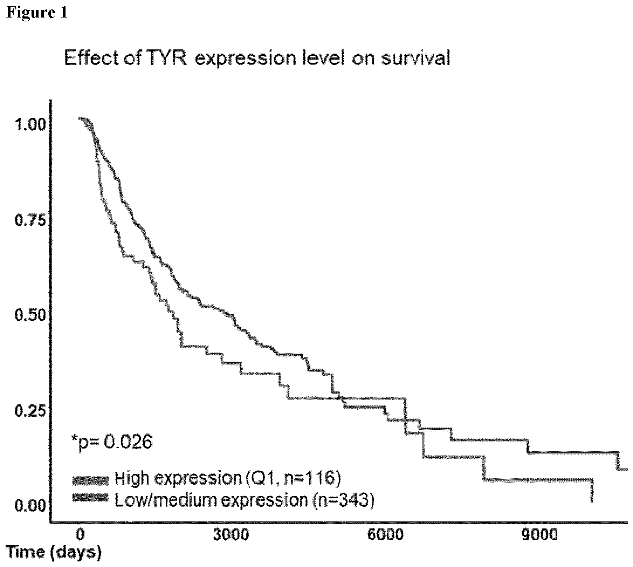

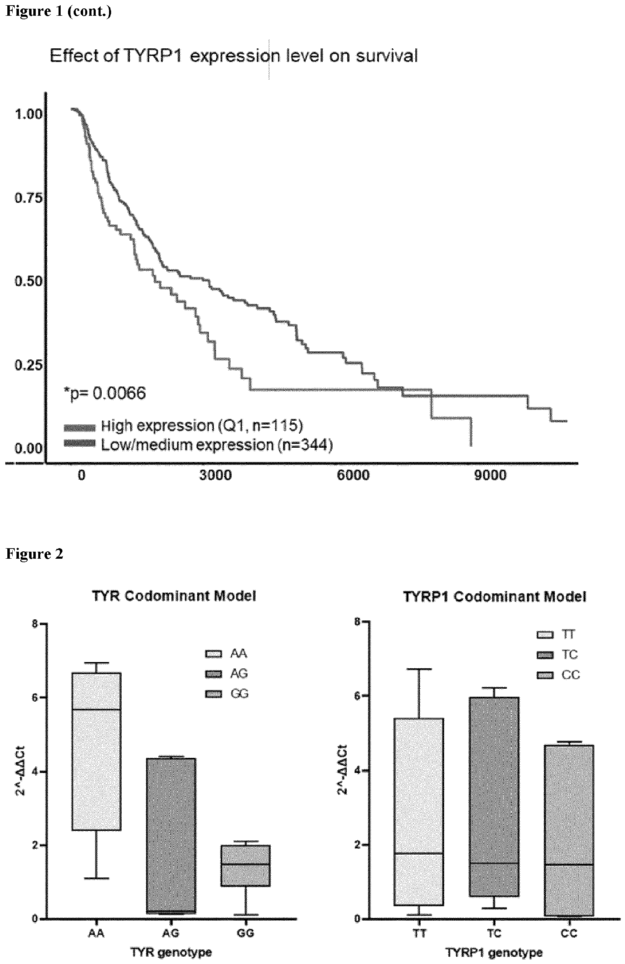

We have not found significant differences among melanoma clinical stages in our target genes. Just in *MTAP* gene we have found statistical differences among stages I and IV (p=0.004). Although we have not indicated any evidence of alteration in gene expression when comparing different clinical states in *TYR* and *TYRP1* genes; we can indicate the existence of differences according to overall survival. Present overall survival analysis showed a higher survival in cutaneous melanoma patients with low/medium *TYR* (p=0.026) and *TYRP1* expression (0.0066), as can be seen in Figure 1. These results reinforce the hypothesis that a high expression of these genes involved in melanogenesis increases melanoma aggressiveness.

### Example 2.5. Functional analysis validation using expression analysis

Two of our characterized SNPs (rs1126809 and rs2733832) were located in genes involved in melanogenesis *(TYR* and *TYRP1,* respectively), and a relation between these genes
expression and cutaneous melanoma patients overall survival was found using *in silico* analyses. We decided to analyze 34 skin samples from our control cohort, and we obtained differential gene expression results according to their genotypes in both SNPs (Figure 2). We could observe significantly higher *TYR* expression values of 2^{-ΔΔCt} in individuals who presented the risk allele in rs1126809 (A) following a codominant model (p-value=0.0133), as we obtained in present meta-analysis data. This association is less clear between *TYRP1* genotypes (rs2733832, risk allele: T, p-value=0.7363), although we can observe the same tendency. According to these and previous data in section 3.4 (TCGA analysis), a high expression pattern of these genes could lead to an increased melanin production and a subsequent worse evolution of the tumor.

### Example 2.6 In silico analysis on the functional and structural impact of the SNPs

Most of the variants changed in intron or upstream/downstream genes, and they do not produce amino acid changes. It is interesting to focus on ENST00000263321.6 in *TYR* gene by its effect on amino acid R/Q produced by a change of G to A.

Functional analysis including the genes of present study *(CLPTM1L, TYR, LOC107987026*, *TYRP1*, *PARP1* and *CTXND2)* shows associations with the risk of suffering MM. *IFNE* and *MTAP* genes were included in this analysis because they are regulated by *LOC107987026* (in addition to the proximity between *MTAP* and *LOC107987026* and their linkage on chromosome 9). Moreover, we included *LURAP1L-AS1* gene, due to its relationship with *TYRP1.*

Data from *DAVID Bioinformatics Resources* confirmed the results of present meta-analysis. According to: i) clinical implication: cancer was found in the first position related diseases in these genes (with 6 associated genes, 75% of the genes and p-value of 9.1^{∗}10⁻⁴). Moreover, MM was associated with 5 genes from the list (62.5% of genes, p-value of 1.6^{∗}10⁻⁸). All these data suggest that present studied genes have a strong relationship with melanoma. ii) Gene function: The most associated terms were "polymorphism" (75% of genes, p-value of 6.9^{∗}10⁻²) and "sequence variation" (75% of genes, p-value of 9.2^{∗}10⁻²). However, as might be expected, an association is also found with "melanin biosynthesis" (25% of genes, p-value of 1.7^{∗}10⁻³) and "melanosome" (25% of genes, p-value 1.0^{∗}10⁻³). iii) Ontology: Genes are mainly involved in the response to chemical and organic substances, especially lipids (62.5% of genes, p-value of 9.2^{∗}10⁻³). This could be explained because some of the proteins encoded by these genes are membrane proteins. iv) Metabolic pathways: The most involved pathways are related to tyrosine metabolism (25% of genes, p-value of 2^{∗}10⁻²) and melanogenesis (25% of genes, p-value of 5.7^{∗}10⁻²).

Moreover, STRING (https://string-db.org/, accessed on 11 June 2021) analysis also reported the strong interaction between *TYR, TYRP1* and *MTAP,* as can be seen in the same cluster; contrasting with remaining genes included in present study. These results reinforce the role of *TYR, TYRP1 and MTAP* as interesting biomarkers of MM.

### Example 2.7. Meta-analysis

Previously published studies have shown similar trends in these SNPs and MM. We have included 11 case-control melanoma studies including previous SNPs to our data analysis. In **Table 4** we represent obtained data with significant differences between cases and controls, including details of HWE.

**Table 4. Summary of the results of present meta-analysis combined with our cohort.**

| SNP | **HWE** | **Significance** | | **OR [95% CI]** |
|---|---|---|---|---|
| **rs45430 (*) (*MX2*)** | 0.7746 | NA | | NA |
| | 0.5092 | | | |
| **rs401681 (*CLPTM1L*)** | 0.9999 | Co dominant Model | 0 | 1.18 [1.12,1.23] |
| | 0.9999 | | | |
| | 0.9999 | | | |
| | 0.9999 | Recessive Model | 4.76 ×10⁻⁸ | 1.23 [1.14,1.32] |
| | 0.9999 | | | |
| | 0.9999 | | | |
| | 0.9999 | Dominant Model | 4 ×10⁻¹⁰ | 1.25 [1.17,1.34] |
| | 0.9999 | | | |
| | 0.9378 | | | |
| **rs1126809 (*TYR*)** | 0.9987 | Codominant Model | 2.72 ×10⁻⁷ | 1.19 [1.11,1.27] |
| | 0.9987 | | | |
| | 0.9987 | Recessive Model | 4.89 ×10⁻⁵ | 1.35 [1.17,1.56] |
| | 0.9987 | | | 1.19 [1.03,1.37] |
| | 0.9987 | Dominant Model | 0.0202 | |
| **rs1335510 (*LOC 107987026*)** | 0.2811 | Co dominant Model | 0.0003 | 1.16 [1.07,1.25] |
| | 0.3948 | Recessive Model | 0.0004 | 1.22 [1.09,1.36] |
| | 0.5107 | Dominant Model | 0.0353 | 1.18 [1.01,1.38] |
| **rs2733832 (*TYRP1*)** | 0.9659 | Co dominant Model | 6.21 ×10⁻⁶ | 1.16 [1.09,1.23] |
| | 0.9845 | Recessive Model | 0.0001 | 1.19 [1.09,1.30] |
| | 0.9659 | Dominant Model | 0.0004 | 1.24 [1.10,1.40] |
| **rs3219090 (PARP1)** | 0.8214 | Co dominant Model | 1.99 ×10⁻⁸ | 1.17 [1.11,1.24] |
| | 0.9982 | | | |
| | 0.9982 | Recessive Model | 5.13 ×10⁻⁷ | 1.20 [1.12,1.29] |
| | 0.9982 | | | |
| | 0.3942 | Dominant Model | 9.23 ×10⁻⁵ | 1.27 [1.13,1.44] |
| | 0.837 | | | |
| **rs7412746 (*CTXND2*)** | 0.9937 | Codominant Model | 1 ×10⁻¹⁰ | 1.19 [1.13,1.26] |
| | 0.9937 | Recessive Model | 1.53 ×10⁻⁸ | 1.26 [1.16,1.37] |
| | 0.9937 | | | |
| | 0.9937 | Dominant Model | 1.4 ×10⁻⁶ | 1.27 [1.15,1.40] |
| **rs7023329 (*) (*MTAP*)** | NA | NA | | NA |

| | | | | |
|---|---|---|---|---|
| *Footnote **Table 4.** (***) This SNP has finally not included because there were not published data fulfilling criteria. NA: not applicable, HWE: Hardy-Weinberg equilibrium, OR: Odds Ratio, 95% CI: Confidence Interval. Codominant Model: A vs a, knowing A is risk allele; Recessive Model: AA vsAa* + *aa; Dominant Model: AA +Aa vs aa* | | | | |

As can be seen in **Table 4**, HWE p-values are all over 0.05, so all data are in HWE. In most of the analysed SNPs show significant differences among cases and controls.

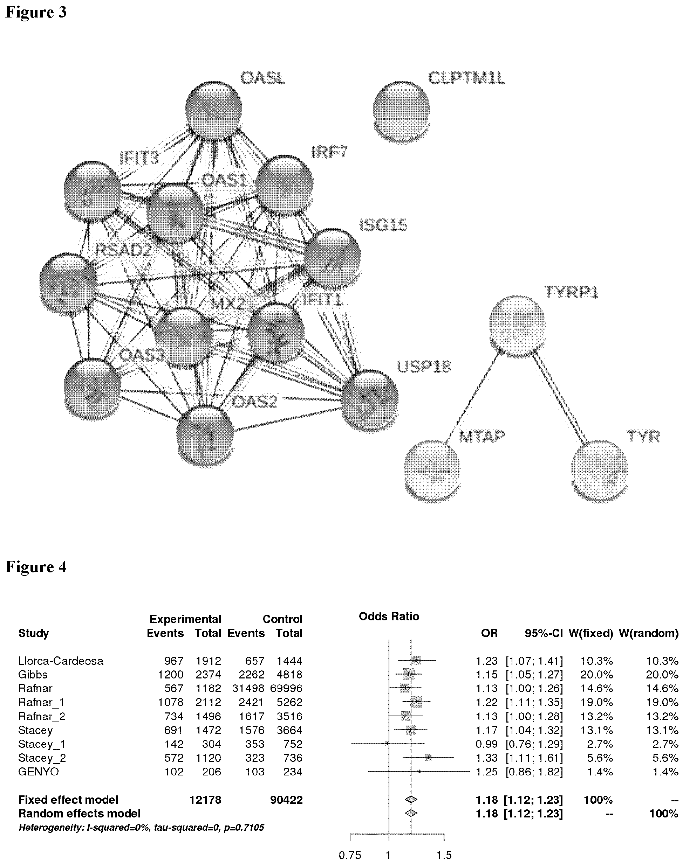

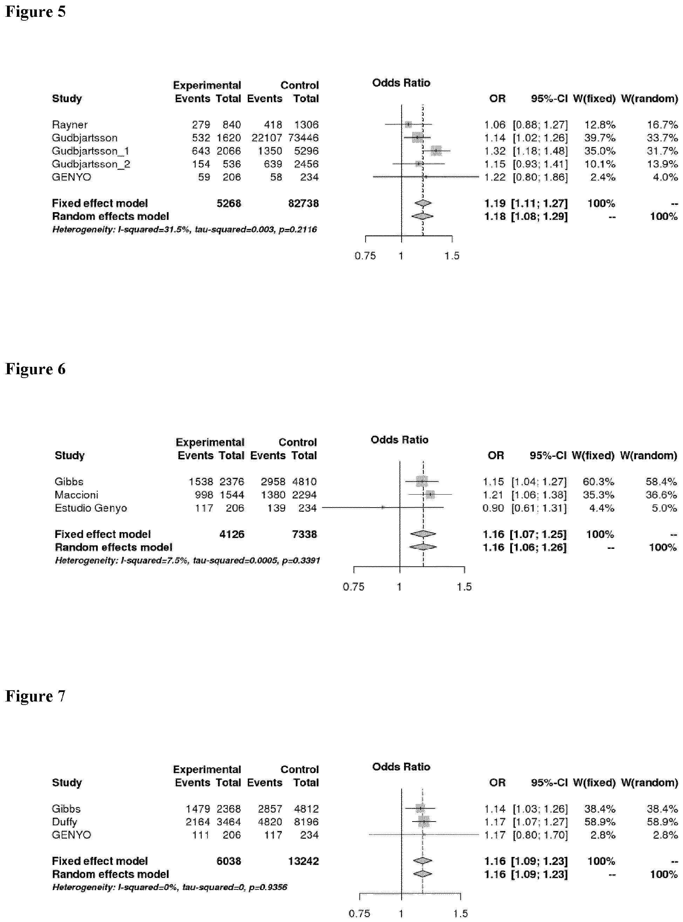

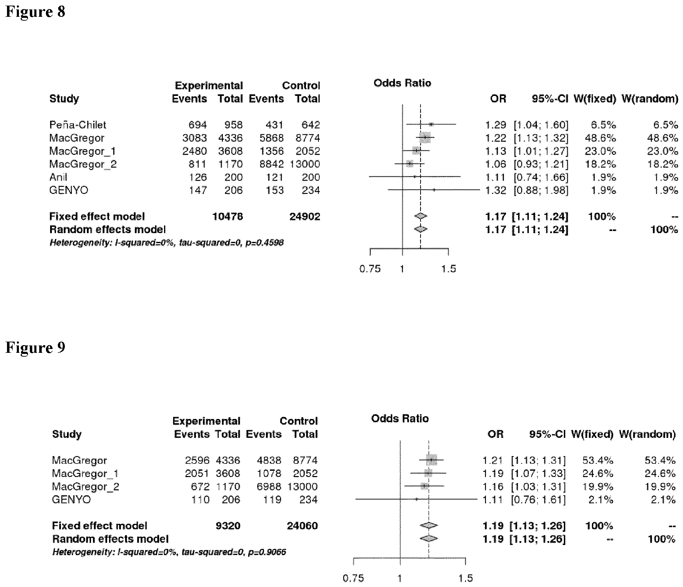

In Figure 4, it is observed the sensibility of the present meta-analysis in rs401681 *(CLPTM1L).* OR from all the articles have similar values, so it means similar data reported in the risk allele and MM. Global OR (1.18) indicates a positive association between the risk allele and MM. In the remaining analysed SNPs (rs1126809, rs1335510, rs2733832, rs3219090 and rs7412746), OR values were over 1 (1.19, 1.16, 1.16, 1.17 and 1.19, respectively), showing the same tendency as in rs401681 (see Figures 5 to 9).

## Claims

1. *In vitro* method for the diagnosis and/or prognosis of malignant melanoma which comprises assessing, in a biological sample obtained from a subject, the presence of at least one allele of the single nucleotide polymorphism (SNP) rs401681 of gene CLPM1L, or the level of expression of the gene CLPM1L having the SNP rs401681, wherein the presence of the SNP rs401681 of gene CLPM1L, or the identification of a higher level of expression of the gene CLPM1L having the SNP rs401681 as compared with a pre-established threshold value, is an indication that the subject is suffering from malignant melanoma or has a bad prognosis.

2. *In vitro* method for determining whether a patient suffering from malignant melanoma is responding to a treatment which comprises assessing, in a biological sample obtained from the patient, the presence of at least one allele of the SNP rs401681 of gene CLPM1L, or the level of expression of the gene CLPM1L having the SNP rs401681, after the administration of the treatment, wherein the presence of the SNP rs401681 of gene CLPM1L, or the identification of a higher level of expression of the gene CLPM1L having the SNP rs401681 as compared with a pre-established threshold value, after the administration of the treatment, is an indication that the subject is not responding to the treatment.

3. *In vitro* method, according to any of the claims 1 or 2, which further comprises assessing the presence of at least one of the following SNPs, or determining the level of expression of the genes having the following SNPs: rs3219090 of gene PARP1, rs2733832 of gene TYRP1, rs1126809 of gene TYR, rs1335510 of gen LOC107987026, and/or rs7412746 of gene CTXND2, or the group consisting of: rs3219090 of gene PARP1, rs2733832 of gene TYRP1, rs1126809 of gene TYR, rs1335510 of gen LOC107987026, and rs7412746 of gene CTXND2.

4. *In vitro* method, according to any of the previous claims, which further comprises determining the level of expression of TYR and/or TYRP1 in a biological sample obtained from the patient, wherein the identification of a higher level of expression as compared with a pre-established threshold value is an indication that the patient is suffering from malignant melanoma, has a bad prognosis or is not responding to the treatment.

5. *In vitro* method, according to any of the previous claims, wherein the diagnosis and/or prognosis of malignant melanoma is confirmed by image techniques, preferably by dermatoscopy.

6. *In vitro* method, according to any of the previous claims, wherein the biological samples is skin tissue, saliva, blood, urine, serum or plasma.

7. *In vitro* method, according to any of the previous claims, wherein the presence of the SNPs is determined by polymerase chain reaction (PCR), preferably real time/quantitative PCR (qPCR) and the level of expression is determined by real time/quantitative PCR (qPCR).

8. *In vitro* use of the SNP rs401681 of gene CLPM1L, or of the gene CLPM1L, for diagnosis and/or prognosis of malignant melanoma, or for determining whether a patient suffering from malignant melanoma is responding to a treatment.

9. *In vitro* use of the SNP rs401681 of gene CLPM1L, or of the gene CLPM1L, according to claim 8, in combination with the use of at least one of the following SNPs or one of the genes having the following SNPs: rs1126809 of gene TYR, rs7412746 of gene CTXND2, rs1335510 of gen LOC107987026, rs2733832 of gene TYRP1 and/or rs3219090 of gene PARP1, for diagnosis and/or prognosis of malignant melanoma, or for determining whether a patient suffering from malignant melanoma is responding to a treatment.

10. *In vitro* use, according to any of the claims 8 or 9, of a group of SNPs or a group of genes having the SNPs consisting of: rs401681 of gene CLPM1L, rs1126809 of gene TYR, rs7412746 of gene CTXND2, rs1335510 of gen LOC107987026, rs2733832 of gene TYRP1 and rs3219090 of gene PARP1, for diagnosis and/or prognosis of malignant melanoma, or for determining whether a patient suffering from malignant melanoma is responding to a treatment.

11. *In vitro* use, according to any of the claims 8 to 10, in combination with image techniques, preferably with dermatoscopy, for the diagnosis and/or prognosis of malignant melanoma, or for determining whether a patient suffering from malignant melanoma is responding to a treatment.

12. Kit consisting of reagents for the identification of the following SNPs or for assessing the level of expression of genes having the following SNPs: rs401681 of gene CLPM1L, rs1126809 of gene TYR, rs7412746 of gene CTXND2, rs1335510 of gen LOC107987026, rs2733832 of gene TYRP1 and rs3219090 of gene PARP1.

13. Use of the kit, according to claim 12, for the diagnosis and/or prognosis of malignant melanoma, or for determining whether a patient suffering from malignant melanoma is responding to a treatment.

14. Anticancer therapy for use in a method for treatment of malignant melanoma in patients which have been diagnosed by means of the method of any of the claims 1 to 7.

15. A computer program or a computer-readable media containing means for carrying out a method as defined in any of the claims 1 to 7.
